# EUROPEAN PATENT APPLICATION

(11) **EP 3 689 189 A1**
(43) Date of publication of application: **05.08.2020**
(21) Application number: 20155011.8
(22) Date of filing: 31.01.2020
(51) Int. Cl.: A47C 27/08, A47C 27/10

(54) **BED APPARATUS CAPABLE OF AUTOMATICALLY ADJUSTING BED SURFACE BASED ON SLEEPING POSITION AND METHOD THEREOF**

(30) Priority: 31.01.2019 TW 108201647 U; 29.11.2019 TW 108143815
(71) Applicant: Green-Sweet Mattress Corp., New Taipei City 239 (TW)
(72) Inventor: LIU, Chih-Yuan, New Taipei City 238 (TW); LIU, Ting-Yu, New Taipei City 238 (TW); LIU, Chung-Han, New Taipei City 238 (TW)
(74) Representative: Lang, Christian

(57) **Abstract**

A bed apparatus capable of automatically adjusting a bed surface based on a sleeping position is disclosed. The bed apparatus includes a multi-layer bed body including an upper layer and a lower layer, material of the upper layer being softer than the material of lower layer; a shoulder airbag fixedly embedded in the upper layer and supported by the lower layer; a waist airbag movably disposed on the upper layer; a sleeping position sensing unit generating a sensing signal in response to the sleeping position of a user; a control box receiving the sensing signal, and determining the sleeping position of the user based on the sensing signal, and controlling the shoulder airbag or the waist airbag to inflate or deflate, responsively.

## Description

### BACKGROUND OF THE INVENTION

### 1. Field of the Invention

The present invention relates to a bed apparatus and a method thereof, and more particularly to a bed apparatus capable of automatically adjusting a height of a bed surface based on a sleeping position, and a method thereof.

### 2. Description of the Related Art

Spinal health is an important factor affecting sleep quality. It is generally believed that the bed supports the sleeping position of the human body, so whether the bed can maintain the height thereof has a certain degree of influence on the spinal health. Because the sleeping position of a user may change during sleep, for example, may change from a lie-on-back sleeping position (that is, the user sleeps on the back thereof) to a lie-on-side sleeping position, or from the lie-on-side sleeping position to lie-on-back sleeping position, and the change of sleeping position will change the support for the vertebrae. When the support for vertebrae is not appropriately adjusted, once the sleeping position is maintained for too long, it will easily cause compression of the spinal nerves or excessive stress on the cervical muscles.

The utility model patent No. M570715 discloses a conventional bed set which can automatically adjust the bed surface thereof according to the sleeping position. The conventional bed set uses a support layer to arrange multiple airbags at the same height, and since the human vertebrae is S-shaped, when the airbag is inflated or deflated, for ergonomic effect, the user's shoulder part needs to be at a lower height and the user's waist part needs to be at a higher height in general. However, when the airbags are set at the same heights in the bed, the inflation height of the airbag corresponding to the waist part can at most reach the surface of the bed only, the waist part of the user having more curved (more S-shaped) lumbar vertebral arch cannot be effectively supported at a height higher than the level of the bed surface, that is, the conventional bed set cannot provide the best support effect for the user's waist part, and cannot provide all users with comprehensive optimal sleep quality.

In addition, the conventional bed set has a plurality of pressure detection modules arranged on a bed surface supported by the airbags, and since the bed surface supported by the airbags generally has softness, the bed surface may be deformed by the user's different sleeping position, and it easily affects the measurement of the pressure detection module, and causes problem of failure of accurate measurement to correctly determine the different sleeping position of the user.

### SUMMARY OF THE INVENTION

An objective of the present invention is to provide a bed apparatus capable of automatically adjusting a bed surface based on a sleeping position and a method thereof, and in the bed apparatus, a sleeping position sensing unit with a support element is used to improve detection of a sleeping position of human body, and the structure of the multi-layer bed body is used to dispose a shoulder airbag and a waist airbag, so as to adjust heights required for supporting a shoulder part and a waist part of the human body in different sleeping position.

In order to achieve the objective, the present invention provides a bed apparatus capable of automatically adjusting a bed surface based on a sleeping position, and the bed apparatus includes a multi-layer bed body, a shoulder airbag, a waist airbag, a sleeping position sensing unit, and a control box. The multi-layer bed body includes an upper layer and a lower layer, and material of the upper layer is softer than material of the lower layer. The shoulder airbag is fixedly embedded in the upper layer and supported by the lower layer. The waist airbag is movably disposed on the upper layer. The sleeping position sensing unit is configured to generate a sensing signal in response to the sleeping position of a user. The control box is configured to receive the sensing signal, and determine the sleeping position of the user based on the sensing signal, and control the shoulder airbag or the waist airbag to inflate or deflate based on the determined sleeping position.

In order to achieve the objective, the present invention provides a method of adjusting a bed surface based on a sleeping position, and the method is applied to a multi-layer bed body which includes an upper layer and a lower layer, and material of the lower layer is harder than material of the upper layer, and the method includes steps: providing a shoulder airbag fixedly embedded in the upper layer and supported by the lower layer; providing a waist airbag movably disposed on the upper layer; providing a sleeping position sensing unit configured to generate a sensing signal in response to the sleeping position of a user on the multi-layer bed body; and determining the sleeping position of the user based on the sensing signal of the sleeping position sensing unit, and controlling the shoulder airbag and the waist airbag to inflate or deflate to adjust heights of the shoulder airbag and the waist airbag based on an adjustment parameter.

According to an embodiment, the control box comprises a pump motor group configured to inflate the shoulder airbag or the waist airbag; a valve group configured to deflate the shoulder airbag or the waist airbag; and a control unit comprising a control panel, and a memory configured to store an adjustment parameter and electrically connected to the pump motor group and the valve group.

According to an embodiment, the control unit receives the sensing signal, and determines the sleeping position of the user based on the sensing signal, and controls the pump motor group and the valve group to adjust heights of the shoulder airbag and the waist airbag based on the adjustment parameter. According to an embodiment, the control unit can adjust the heights of the shoulder airbag and the waist airbag in response to an operation of the control panel, and an operation result of the control panel is stored in the memory as the adjustment parameter.

According to an embodiment, the upper layer has a multi-layer structure, and the waist airbag is movably disposed in the multi-layer structure of the upper layer; or, the waist airbag is movably disposed on an upper surface of the upper layer, or between the upper layer and the lower layer.

According to an embodiment, the sleeping position sensing unit is disposed between the shoulder airbag and the waist airbag and on a horizontal plane of the multi-layer bed body.

According to an embodiment, the sleeping position sensing unit comprises a strip-shaped support body, and a plurality of pressure sensors distributed and disposed on the strip-shaped support body and electrically connected to the control box.

According to an embodiment of the bed apparatus capable of automatically adjusting bed surface based on sleeping position and the method according to the present invention, a multi-layer bed body includes an shoulder airbag fixedly embedded therein, and a waist airbag movably disposed thereon, so that when a user lies on the multi-layer bed body, a shoulder part and a waist part of the user can be supported optimally in response to different sleeping position of the user, thereby greatly improving the user's sleep quality, especially for the user having more curved lumbar vertebral arch, that is, the shape of the user's lumbar vertebral arch is more like a S shape. In addition, compared with the conventional bed set which needs to use more airbag, the bed apparatus of the present invention only needs to use the shoulder airbag and the waist airbag, so the production cost of the bed apparatus of the present invention can be relatively low.

### BRIEF DESCRIPTION OF THE DRAWINGS

The structure, operating principle and effects of the present invention will be described in detail by way of various embodiments which are illustrated in the accompanying drawings.
FIG. 1 is a block diagram of a bed apparatus of the present invention.
FIG. 2 is a top view of a user lying on a bed apparatus of the present invention.
FIGS. 3A to 3D are sectional views of a multi-layer bed body of a bed apparatus of the present invention.
FIG. 4 is a sectional view of a sleeping position sensing unit of a bed apparatus of the present invention.
FIG. 5 is a flowchart of an operation of a bed apparatus automatically adjusting a bed surface based on a sleeping position, according to the present invention.

### DETAILED DESCRIPTION OF THE PREFERRED EMBODIMENTS

The following embodiments of the present invention are herein described in detail with reference to the accompanying drawings. These drawings show specific examples of the embodiments of the present invention. These embodiments are provided so that this disclosure will be thorough and complete, and will fully convey the scope of the invention to those skilled in the art. It is to be acknowledged that these embodiments are exemplary implementations and are not to be construed as limiting the scope of the present invention in any way. Further modifications to the disclosed embodiments, as well as other embodiments, are also included within the scope of the appended claims. These embodiments are provided so that this disclosure is thorough and complete, and fully conveys the inventive concept to those skilled in the art. Regarding the drawings, the relative proportions and ratios of elements in the drawings may be exaggerated or diminished in size for the sake of clarity and convenience. Such arbitrary proportions are only illustrative and not limiting in any way. The same reference numbers are used in the drawings and description to refer to the same or like parts. As used herein, the term "or" includes any and all combinations of one or more of the associated listed items.

It will be acknowledged that when an element or layer is referred to as being "on," "connected to" or "coupled to" another element or layer, it can be directly on, connected or coupled to the other element or layer, or intervening elements or layers may be present. In contrast, when an element is referred to as being "directly on," "directly connected to" or "directly coupled to" another element or layer, there are no intervening elements or layers present.

In addition, unless explicitly described to the contrary, the word "comprise", "include" and "have", and variations such as "comprises", "comprising", "includes", "including", "has" and "having" will be acknowledged to imply the inclusion of stated elements but not the exclusion of any other elements.

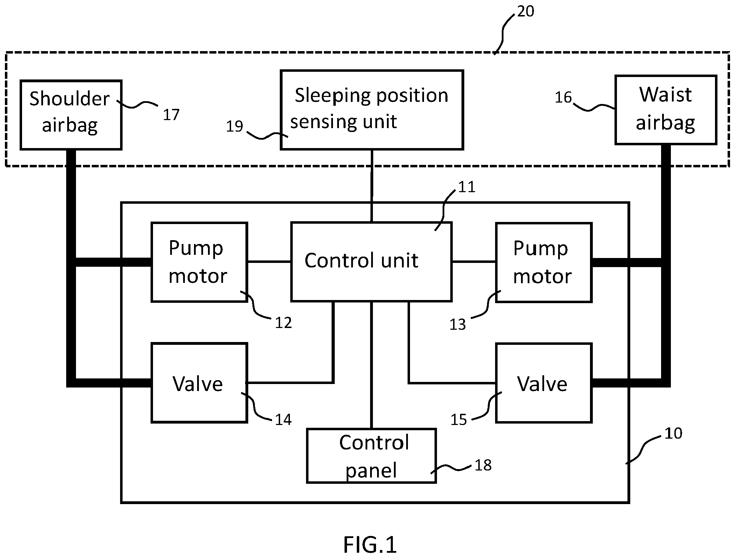

Please refer to FIG. 1, which shows a block diagram of a bed apparatus of the present invention. In an embodiment of the present invention, the bed apparatus capable of automatically adjusting a bed surface based on a sleeping position comprises a control box 10, a shoulder airbag 17, a waist airbag 16 and a sleeping position sensing unit 19. The shoulder airbag 17 is a vertical airbag, the waist airbag 16 is a flat airbag, the sleeping position sensing unit 19 includes a plurality of pressure sensors (not shown in FIG. 1), and the control box 10 is electrically connected to the pressure sensors of the sleeping position sensing unit 19 and configured to receive a plurality of pressure sensing signals. The control box 10 is in communication with the shoulder airbag 17 and the waist airbag 16, to inflate or deflate the shoulder airbag 17 and the waist airbag 16 to adjust heights of the shoulder airbag 17 and the waist airbag 16. In an embodiment of the present invention, the shoulder airbag 17 is in a rectangular shape, and the waist airbag 16 is in an elliptic or semicircular shape. A plurality of support spacers (not shown in FIG. 1) are disposed in an interior space of each of the shoulder airbag 17 and the waist airbag 16, to make these airbags maintain original shapes thereof without deformation after inflation or deflation.

The control box 10 of the bed apparatus includes a pump motor group and a valve group. The pump motor group includes two pump motors 12 and 13 configured to inflate the shoulder airbag 17 and the waist airbag 16, respectively. The valve group includes two valves 14 and 15 configured to deflate the shoulder airbag 17 and the waist airbag 16, respectively. The shoulder airbag 17 is in communication with the pump motor 12 and the valve 14, the waist airbag 16 is in communication with the pump motor 13 and the valve 15; the pump motors 12 and 13 and the valves 14 and 15 are electrically connected to a control unit 11, so that the control unit 11 can be used to control operations of the pump motors 12 and 13 and the valves 14 and 15.

The control unit 11 of the control box 10 of the present invention includes a memory (not shown in FIG. 1), and the control unit 11 is electrically connected to the sleeping position sensing unit 19 and a control panel 18, and the control panel 18 is connected to a remote controller (not shown in FIG. 1) by a wireless communication manner. The control unit 11 can receive a plurality of pressure sensing signals of the pressure sensors from the sleeping position sensing unit 19, and determine an adjustment parameter of the sleeping position based on the pressure sensing signals and store the adjustment parameter in the memory. In response to the operation of the control panel 18 by the remote controller, the control unit 11 can control the operations of the pump motors 12 and 13 and the valves 14 and 15, to adjust heights of the shoulder airbag 17 and the waist airbag 16, and store the operation result of the control panel 18 in the memory as a part of the adjustment parameter. Furthermore, the control box 10 of the present invention further includes two air pressure sensors (not shown in FIG. 1) electrically connected to the control unit 11 and configured to sense pressures of the shoulder airbag 17 and the waist airbag 16, respectively. The operation result of the control panel 18 can include the operations of adjusting the pressures of the shoulder airbag 17 and the waist airbag 16 in response to different sleeping position, and controlling electrical characteristics of the pump motor group and the valve group in response to different sleeping position.

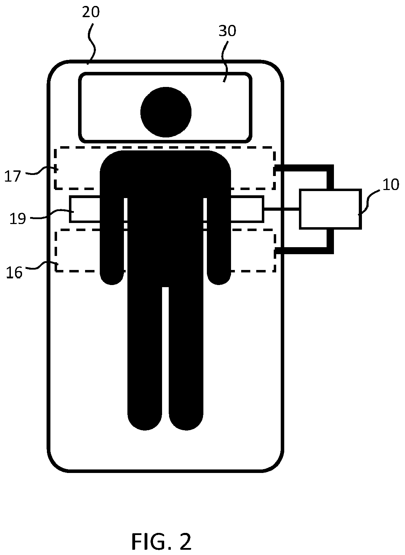

Please refer to FIG. 2, which shows a top view of a user lying on the bed apparatus of the present invention. In an embodiment of the present invention, the multi-layer bed body 20 includes a shoulder airbag 17, a waist airbag 16 and a sleeping position sensing unit 19 disposed in the inside thereof. As shown in the top view of the multi-layer bed body 20 in FIG. 2, the shoulder airbag 17 is disposed adjacent to a pillow 30, the waist airbag 16 is movably disposed in response to a waist position of a user, and the sleeping position sensing unit 19 is disposed between the shoulder airbag 17 and the waist airbag 16 on a horizontal plane of the multi-layer bed body 20. When the user lies on the bed surface of the multi-layer bed body 20, as shown in FIG. 2, the shoulder airbag 17 and the waist airbag 16 can appropriately support a shoulder part and a waist part of the user in a sleeping position, and the sleeping position sensing unit 19 is located at an appropriate position to sense a lie-on-back sleeping position and a lie-on-side sleeping position of the user.

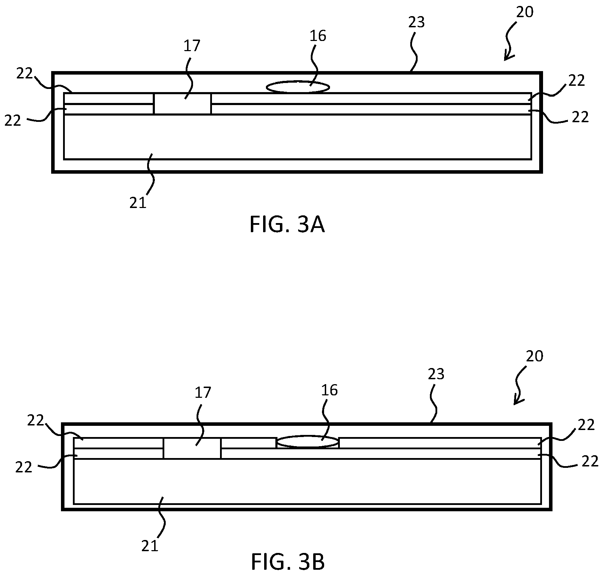

Please refer to FIGS. 3A to 3D, which show sectional views of a multi-layer bed body of a bed apparatus of the present invention. In various embodiments of the present invention, the multi-layer bed body 20 is in a layered structure and includes an upper layer and a lower layer. The lower layer is a support layer 21, and the upper layer is an adjustment layer which can be a multi-layer foam structure 22 or a layer of thicker foam structure 22; therefore, material of the upper layer is softer than material of the lower layer. The multi-layer bed body 20 can further include a bottom sheet 23 configured to accommodates the layered structure formed by the upper layer and the lower layer. The two-layer foam structure 22 is used as an example of the upper layer of the multi-layer bed body 20 for illustrations of various embodiments of the present invention.

In various embodiments of the present invention, the multi-layer bed body 20 can includes the bottom sheet 23 configured to accommodate the support layer 21 and two layers of foam structures 22, a bed surface of the bottom sheet 23 has a comfort layer which can be a thick layer or a thin layer. The shoulder airbag 17 is fixedly embedded between the two layers of foam structures 22 of the upper layer and adjacent to a bottom of the pillow 30. Preferably, the shoulder airbag 17 can be a vertical airbag and have a maximum inflation height of 5 cm. An inflation height of the shoulder airbag 17 is equal to or lower than a height of the upper layer, that is, the inflation height of the shoulder airbag 17 is at most the same as the height of the upper layer.

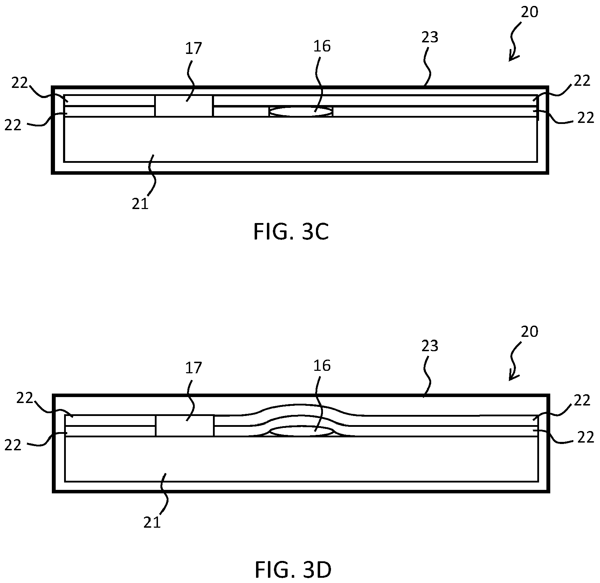

In addition, correspondingly in position to a waist position of the user, the waist airbag 16 can be attached with an upper surface of the two layers of foam structures 22, as shown in FIG. 3A, or the waist airbag 16 can also be embedded in the upper one of two layers of the foam structure 22, as shown in FIG. 3B, or the waist airbag 16 can also be embedded in the lower one of two layers of the foam structure 22, as shown in FIG. 3C, or the waist airbag 16 can also be attached with a lower surface of the two-layer foam structure 22, that is, between the support layer 21 and the two-layer foam structure 22, as shown in FIG. 3D. Preferably, the waist airbag 16 can be a flat airbag and have a maximum inflation height of 3.5 cm. In addition, in the embodiment shown in FIGS. 3A to 3D, the two layers of foam structures 22 can be replaced by a layer of thicker foam structure 22.

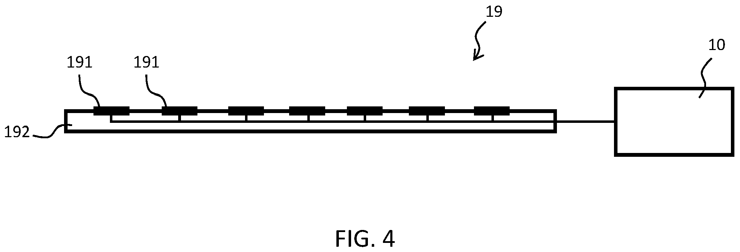

Please refer to FIG. 4, which show a sectional view of a sleeping position sensing unit of a bed apparatus of the present invention. In an embodiment of the present invention, in order to solve the problem that the bed surface may be deformed, because of different sleeping position of user, to easily affect measurement accuracy of the pressure sensor, the sleeping position sensing unit 19 of the present invention includes a strip-shaped support body 192 disposed between the shoulder airbag 17 and the waist airbag 16 on the horizontal plane of the multi-layer bed body 20. A plurality of pressure sensors 191 are disposed on an upper surface of the strip-shaped support body 192 and electrically connected to the control unit 11 of the control box 10. With the disposal of the support body, the measurement of the pressure sensor 191 is not affected by the deformation of the bed surface when the user lies on the bed surface, regardless if the sleeping position sensing unit 19 is disposed on the upper surface of the multi-layer foam structure 22 or sandwiched between the two layers of foam structures 22, thereby improving the accuracy of the control unit 11 to determine the sleeping position of the user. In an embodiment of the present invention, one or more of the plurality of pressure sensors can be a pressure sensor with high sensing sensitivity, and the pressure sensor with high sensing sensitivity can be used to sense slight body movement caused by breathing or heartbeat when the user is sleeping, thereby obtaining a sleep quality index based on the sensing results of the sleeping position and the slight body movement, and the sleep quality index can be used as the basis for determining sleep disorders such as sleep apnea.

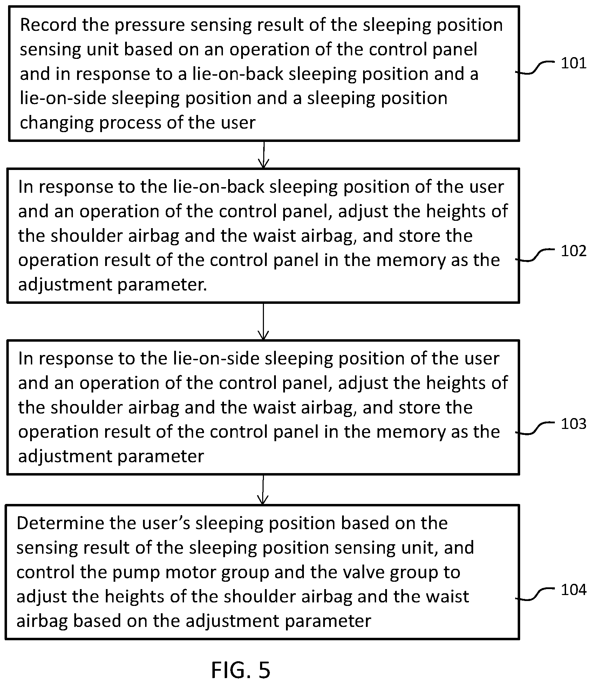

Please refer to FIG. 5, which shows a flowchart of an operation of the bed apparatus automatically adjusting a bed surface based on a sleeping position, according to the present invention. In an embodiment of the present invention, a method of adjusting the bed surface based on the sleeping position can be applied to the multi-layer bed body 20 of the bed apparatus shown in FIG. 1, and the method includes steps 101 to 104. In a step 101, when a user lies on the bed surface of the multi-layer bed body 20, the control unit 11 records and stores the pressure sensing result of the sleeping position sensing unit 19 in the memory as the adjustment parameter based on an operation of the control panel and in response to a lie-on-back sleeping position and a lie-on-side sleeping position and a sleeping position changing process of the user, respectively.

In an embodiment of the present invention, during the sleeping position changing process when the user lies and sleeps on the multi-layer bed body 20, when the user is in a lie-on-side sleeping position, the sleeping position sensing unit 19 can sense one maximum pressure value, and when the user is in a lie-on-back sleeping position, the sleeping position sensing unit 19 can sense two maximum pressure values, so that the sleeping position sensing unit 19 can determine whether the user is in the lie-on-back sleeping position (that is, the user sleeps on the back thereof) or the lie-on-side sleeping position (that is, the user sleeps on the side thereof), based on the number of the sensed maximum pressure values, and the control unit 11 can control the pump motor group or the valve group to inflate or deflate the shoulder airbag 17 and the waist airbag 16 based on the determination result of the sleeping position and parameter settings, thereby increasing or decreasing the pressure applied on the shoulder part or the waist part of the user to a most appropriate pressure P'.

The parameter settings include physiological or non-physiological parameters related to the user, such as height, weight, age, gender, and so on. The parameter settings can be set by the manufacturer, or by the user through the operation of the control panel 18, so as to store the adjustment parameter in the memory. Therefore, after the control unit 11 determines that the user is in the lie-on-back sleeping position (that is, the user sleeps on the back thereof) or the lie-on-side sleeping position (that is, the user sleeps on the side thereof), the airbag 17 and 16 can be adjusted to the optimal hardness degree based on the physiological or non-physiological parameters related to the user, so that the pressure applied to the shoulder part or the waist part of the user can be adjusted to the optimal pressure. The storage of the adjustment parameter in the step 102 and 103 is further explained below.

In a step 102, in response to the lie-on-back sleeping position of the user and an operation of the control panel 18, the control unit 11 controls the pump motor group and the valve group to inflate or deflate the shoulder airbag 17 and the waist airbag 16, so as to adjust the heights of the shoulder airbag 17 and the waist airbag 16 to appropriately support the shoulder part and the waist part of the user in the lie-on-back sleeping position, and the operation result of the control panel 18 is stored in the memory as the adjustment parameter. When the user is in the lie-on-back sleeping position, the required deflation degree of the shoulder airbag 17 is lower, so the height of the shoulder airbag 17 in the lie-on-back sleep position is higher than that in the lie-on-side sleep position. The operation of the control panel 18 must be determined based on the curved degree of the lumbar vertebral arch of the user, and when the user's lumbar vertebral arch is more curved, that is, the shape of the user's lumbar vertebral arch is more like an S shape, the required inflation degree of the waist airbag 16 is higher, that is, the inflation height of the waist airbag 16 is higher; when the user's lumbar vertebral arch is less curved, that is, the shape of the user's lumbar vertebral arch is less like an S shape, the required inflation degree of the waist airbag 16 is lower, that is, the inflation height of the waist airbag 16 is lower.

In a step 103, in response to the lie-on-side sleeping position of the user and an operation of the control panel 18, the control unit 11 controls the pump motor group and the valve group to inflate or deflate the shoulder airbag 17 and the waist airbag 16, to adjust the heights of the shoulder airbag 17 and the waist airbag 16 to appropriately support the shoulder part and the waist part of the user in the lie-on-side sleeping position, and store the operation result of the control panel 18 in the memory as the adjustment parameter. When the user is the lie-on-side sleeping position, the required deflation degree of the shoulder airbag 17 is higher, so the height of the shoulder airbag 17 in the lie-on-side sleep position is lower than that in the lie-on-back sleep position, the operation of the control panel 18 must be determined based on the user's side waist, and when the waist of the user is more obvious, the required inflation degree of the waist airbag 16 is higher, that is, the inflation height of the waist airbag 16 is higher; when the waist of the user is not obvious, for example, the user has bucket waist, the required inflation degree of the waist airbag 16 is lower, that is, the inflation height of the waist airbag 16 is lower.

In a step 104, after the steps 101, 102 and 103, a set of adjustment parameters, which is related to determination of the pressure sensing result corresponding to the sleeping position and the electrical characteristics for controlling the pump motor group and the valve group to inflate or deflate the shoulder airbag 17 and the waist airbag 16 in response to the sleeping position of the user, is stored in the memory of the control unit 11.

When the user lies and sleeps on the multi-layer bed body 20, the control unit 11 can monitor the pressure sensing result of the sleeping position sensing unit 19 in real time, and determine the current sleeping position of the user based on the adjustment parameter for determining the pressure sensing result of the sleeping position. Based on the determined sleeping position, the control unit 11 reads the corresponding electrical characteristics stored in the memory and for controlling the pump motor group and the valve group, and then controls the pump motor group and the valve group to inflate or deflate the shoulder airbag 17 and the waist airbag 16 based on the read electrical characteristics, so as to adjust the heights and the optimized hardness degrees of the shoulder airbag 17 and the waist airbag 16, thereby adjusting the pressure applied to the shoulder part or the waist part of the user to the most appropriate pressure based on the determined sleeping position.

The present invention disclosed herein has been described by means of specific embodiments. However, numerous modifications, variations and enhancements can be made thereto by those skilled in the art without departing from the spirit and scope of the disclosure set forth in the claims.

## Claims

1. A bed apparatus capable of automatically adjusting a bed surface based on a sleeping position, comprising:
a multi-layer bed body comprising an upper layer and a lower layer, wherein material of the upper layer is softer than material of the lower layer;
a shoulder airbag fixedly embedded in the upper layer and supported by the lower layer;
a waist airbag movably disposed on the upper layer;
a sleeping position sensing unit configured to generate a sensing signal in response to the sleeping position of a user; and
a control box configured to receive the sensing signal, and determine the sleeping position of the user based on the sensing signal, and control the shoulder airbag or the waist airbag to inflate or deflate based on the determined sleeping position.

2. The bed apparatus according to claim 1, wherein the control box comprises:
a pump motor group configured to inflate the shoulder airbag or the waist airbag;
a valve group configured to deflate the shoulder airbag or the waist airbag; and
a control unit comprising a memory configured to store an adjustment parameter and electrically connected to the pump motor group and the valve group.

3. The bed apparatus according to claim 2, wherein the control unit receives the sensing signal, and determines the sleeping position of the user based on the sensing signal, and controls the pump motor group and the valve group to adjust heights of the shoulder airbag and the waist airbag based on the adjustment parameter.

4. The bed apparatus according to claim 2, wherein the control box further comprises a control panel electrically connected to the control unit, and configured to enable the control unit to adjust heights of the shoulder airbag and the waist airbag in response to an operation of the control panel, and an operation result of the control panel is stored in the memory as the adjustment parameter.

5. The bed apparatus according to claim 1, wherein the multi-layer bed body, the shoulder airbag, and the waist airbag are accommodated in a bottom sheet.

6. The bed apparatus according to claim 1, wherein the upper layer has a multi-layer structure, and the waist airbag is movably disposed in the multi-layer structure of the upper layer.

7. The bed apparatus according to claim 1, wherein the waist airbag is movably disposed on an upper surface of the upper layer, or between the upper layer and the lower layer.

8. The bed apparatus according to claim 1, wherein the sleeping position sensing unit is disposed between the shoulder airbag and the waist airbag and on a horizontal plane of the multi-layer bed body.

9. The bed apparatus according to claim 1, wherein the sleeping position sensing unit comprises a strip-shaped support body, and a plurality of pressure sensors distributed and disposed on the strip-shaped support body and electrically connected to the control box.

10. A method of adjusting a bed surface based on a sleeping position, applied to a multi-layer bed body, wherein the multi-layer bed body comprises an upper layer and a lower layer, and material of the lower layer is harder than material of the upper layer, and the method comprises:
providing a shoulder airbag fixedly embedded in the upper layer and supported by the lower layer;
providing a waist airbag movably disposed on the upper layer;
providing a sleeping position sensing unit configured to generate a sensing signal in response to the sleeping position of a user on the multi-layer bed body; and
determining the sleeping position of the user based on the sensing signal of the sleeping position sensing unit, and controlling the shoulder airbag and the waist airbag to inflate or deflate to adjust heights of the shoulder airbag and the waist airbag based on an adjustment parameter.

11. The method according to claim 10, wherein the sleeping position sensing unit is disposed between the shoulder airbag and the waist airbag and on a horizontal plane of the multi-layer bed body.

12. The method according to claim 10, wherein the upper layer has a multi-layer structure, and the waist airbag is movably disposed in the multi-layer structure of the upper layer.

13. The method according to claim 10, further comprising:
movably disposing the waist airbag on an upper surface of the upper layer, or between the upper layer and the lower layer.

14. The method according to claim 10, further comprising:
providing a control box having a control panel, and using the control box to inflate or deflate the shoulder airbag and the waist airbag based on an operation of the control panel.

15. The method according to claim 14, wherein in response to a lie-on-back sleeping position or a lie-on-side sleeping position of the user, the control box adjusts heights of the shoulder airbag and the waist airbag based on an operation of the control panel, so as to generate the adjustment parameter and store a sensing result of the sleeping position sensing unit and the adjustment parameter.
